# EUROPEAN PATENT APPLICATION

(11) **EP 4 442 833 A1**
(43) Date of publication of application: **09.10.2024**
(21) Application number: 22912056.3
(22) Date of filing: 26.12.2022
(51) Int. Cl.: C12P 13/22, C12P 13/08, C12P 13/06, A23K 20/142, A23K 10/16

(54) **METHOD FOR PREPARING AMINO ACID-CONTAINING PRODUCT FROM FERMENTED SOLUTION**

(30) Priority: 24.12.2021 KR 20210187593
(71) Applicant: CJ CheilJedang Corporation, Seoul 04560 (KR)
(72) Inventor: KWON, Min Kyung, Seoul 04560 (KR); SHIN, Jihyun, Seoul 04560 (KR); KIM, Jong Hyun, Seoul 04560 (KR); PARK, Sang Min, Seoul 04560 (KR); KANG, Ji-hun, Seoul 04560 (KR); KIM, Min Sup, Seoul 04560 (KR)
(74) Representative: Regimbeau
(86) International application number: PCT/KR2022/021270
(87) International publication number: WO 2023/121423

(57) **Abstract**

The present application relates to a method for preparing amino acid granules from a fermentation broth, wherein the prepared amino acid granules contain no cells.

## Description

### [Technical Field]

The present application relates to a method for preparing amino acid granule, from which cells are removed, from a fermentation broth.

### [Background Art]

As one of the methods for producing amino acid granules for feed, a fermentation method using microorganisms is used, and amino acid granules can be produced by directly drying and solidifying a fermentation broth of microorganisms.

In particular, a method for granulating mixed amino acids with low solubility is known in the art (US 2021-0094903 A1).

When preparing an amino acid granule from the fermentation broth, strains (cells) used in the fermentation broth production process may remain in the fermentation broth. When cells are contained in an amino acid granule, there is a problem in that the product registration process becomes complicated, and it is difficult to sell the product in countries with GM strain issues. In addition, in the case of granules, there is an issue in that it is difficult to produce a product with a purity higher than that of the fermentation broth, but by removing the cells, there is an advantage in that a product with a higher content than conventional granules can be produced.

### [Prior Art Literature]

### [Patent Literature]

(Patent Literature 1) U.S. Application Publication No. 2021-0094903 A1
(Patent Literature 2) U.S. Patent No. 8465962 B2
(Patent Literature 3) U.S. Patent No. 9885093 B2
(Patent Literature 4) U.S. Patent No. 10351859 B2
(Patent Literature 5) U.S. Patent No. 7863435 B2
(Patent Literature 6) U.S. Patent No. 10787692 B2
(Patent Literature 7) U.S. Patent No. 9029105 B2
(Patent Literature 8) U.S. Application Publication No. 2021-0094903 A1

### [Disclosure]

### [Technical Problem]

One object of the present application is to provide a method for preparing a granule product having a high amino acid content and a uniform content for each particle size compared to conventional fermented broth and having fewer GM strain issues than conventional granule products due to the removal of cells.

In addition, another object of the present application is to provide an amino acid granule having a high amino acid content compared to conventional fermentation broth and having fewer GM strain issues than conventional granule products due to the removal of cells.

### [Technical Solution]

The present application will be described in detail as follows. Meanwhile, each description and embodiment disclosed in this disclosure may also be applied to other descriptions and embodiments. That is, all combinations of various elements disclosed in this disclosure fall within the scope of the present application. Further, the scope of the present application is not limited by the specific description below.

Additionally, a number of papers and patent literature references have been cited throughout the present specification. The content of the cited papers and patent literature references is incorporated herein by reference in its entirety, and the level of the technical field to which the present invention belongs and the content of the present invention will be described more clearly.

In order to achieve the objects above, one aspect of the present application provides a method for preparing an amino acid granule, including:
a first step of preparing a fermentation filtrate by removing cells from a fermentation broth containing amino acids; and
a second step of forming granules from the fermentation filtrate.

The preparation method of the present application simplifies the product registration process by removing cells in the process of preparing amino acid granules, and can deal in advance with countries with genetically modified (GM) issues. In addition, in the case of granules, there is an issue that it is difficult to produce a product with a purity higher than that of the fermentation broth, but there is an advantage in that a product with a higher content than conventional granules can be produced by removing cells. Therefore, product purity can be diversified by using the preparation method of the present application.

As used herein, the term "amino acid granule" refers to a mixture containing amino acids in the form of granules. However, if necessary, the formulation of the amino acid granule may vary as long as the spirit of the invention of the present application is not changed. Additionally, as described above, subsequent processes may be further performed to implement formulations of various amino acid granules. The above-mentioned amino acid granule can be used as an additive for animal feed, *etc.*, and the use thereof is not limited.

Hereinafter, each step of the method for preparing an amino acid granule according to one aspect of the present application will be described in detail.

First, a first step of preparing a fermentation filtrate by removing cells from a fermentation broth containing amino acids is performed.

As used herein, the term "the removal of cells" is not limited in that the cells are not contained at all in a solution or a product. Specifically, the removal of the cells from the solution or product means that the strain used to prepare the amino acid fermentation broth was isolated from the solution or product to the extent that the mass could be neglected. Therefore, in analyzing the mass of the fermentation filtrate, which is a fermentation broth from which the cells are removed, or the amino acid granule prepared therefrom, and the composition of the product, the mass occupied by the cells can be neglected.

The amino acid contained in the fermentation broth of the first step may be an L-amino acid, and in particular, the amino acid may be an amino acid with low solubility. For example, the amino acid to which the preparation method of the present application can be applied may be an amino acid having a low solubility of greater than 0 g/100 g and less than or equal to 20 g/100 g in water at 25°C, but is not limited thereto. By applying the amino acid with low solubility as described above, a more remarkable effect can be expected compared to the existing process.

Specifically, the amino acid may be valine, tryptophan, threonine, isoleucine, leucine, methionine, histidine, or phenylalanine, but is not limited thereto.

The amino acid contained in the fermentation broth of the first step may be prepared by an extraction method, a synthesis method, a fermentation method, an enzyme method, *etc.* In some cases, the amino acid may be provided from a fermentation product prepared by a fermentation method using a microorganism. As used herein, the term "fermentation product" or "fermentation broth" refers to a product of an enzymatic or metabolic synthesis or degradation of organic materials using microorganisms. For example, it may include the culture itself containing the microorganism obtained by culturing the microorganism in a culture medium, or a concentrate, a dried product, or a freeze-dried product of a culture obtained by removing the microorganism therefrom. In this case, the amino acid mixture solution may include L-amino acids and the entire fermentation product, or may have impurities removed from the fermentation product. In the present application, the fermentation broth containing the amino acid may be a liquid of a fermentation product obtained by the fermentation methods disclosed in US 8465962 B2, US 9885093 B2, US 10351859 B2, US 7863435 B2, US 10787692 B2, US 9029105 B2, and US 2021-0094903 A1, but is not limited thereto.

The "fermentation broth containing amino acids" of the present application may be used interchangeably with "amino acid-containing fermentation broth" or "amino acid fermentation broth".

The fermentation broth prepared to provide amino acids in the first step may be a result obtained by culturing a microorganism that produces L-amino acids. At this time, as used herein, the term "microorganism producing L-amino acids" includes both wild-type microorganisms and microorganisms in which genetic modification has occurred naturally or artificially, and it may be a microorganism including genetic modification for the production of a desired protein or product as a microorganism in which specific mechanism is weakened or enhanced as an external gene is inserted or the activity of an endogenous gene is enhanced or inactivated, *etc.*

The above-described microorganism used in the first step may be at least one selected from a microorganism of the genus *Brevibacterium*, the genus *Corynebacterium,* the genus *Escherichia*, the genus *Serratia,* the genus *Erwinia*, the genus *Enterobacteria*, the genus *Streptomyces,* or the genus *Pseudomonas*, *etc.,* or artificial mutants thereof. Therefore, the first step may further include the step of preparing an amino acid mixed solution using at least one of the microorganisms described above.

Among the above-mentioned microorganisms of the present application that can be used in the first step, "the microorganism of the genus *Corynebacterium*" may include all microorganisms of the genus *Corynebacterium.* Specifically, it may be *Corynebacterium glutamicum*, *Corynebacterium crudilactis*, *Corynebacterium deserti*, *Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* and more specifically *Corynebacterium glutamicum,* but is not limited thereto.

The microorganisms of the genus Corynebacterium, particularly *Corynebacterium glutamicum*, are Gram-positive microorganisms that are widely used in the production of L-amino acids and other useful substances. In order to produce the L-amino acids and other useful substances, various studies are being conducted to develop microorganisms with high-efficiency production and technologies for fermentation processes. For example, in a microorganism of the genus *Corynebacterium*, a target material-specific approach such as increasing the expression of a gene encoding an enzyme involved in biosynthesis of L-tryptophan, L-valine, L-isoleucine, L-leucine, L-histidine, or L-threonine, *etc.*, or removing a gene unnecessary for the biosynthesis is mainly used.

In the first step, the cells are removed from the prepared fermentation broth as described above. The removal of cells does not necessarily refer to removing the cells completely from the fermentation broth to make the fermentation broth sterile as described above. In order to remove the cells, centrifugation, filtration, treatment with a protein crystallizing precipitant (salting-out method), extraction, ultrasonic disruption, ultrafiltration, dialysis, various kinds of chromatography such as molecular sieve chromatography (gel filtration), adsorption chromatography, ion exchange chromatography, affinity chromatography, *etc.*, HPLC, or a combination thereof may be used. In addition to the methods described above, the cells can be separated from the fermentation broth using a suitable method known in the art.

In the first step, in some cases, a step of separating amino acid crystals from the fermentation broth or dissolving the amino acid crystals in the fermentation broth may be performed prior to isolating the cells. The amino acid crystals mean that some amino acids are crystallized in the fermentation broth when a high concentration of amino acids having relatively low solubility is contained in the fermentation broth. When the solution contains amino acid crystals, there is a concern that the amino acid crystals may also be removed during the cell removal process. The amino acid crystals can be recycled by dissolving in the fermentation broth or separately isolating them prior to cell removal, and thus amino acids produced in the fermentation process can be prevented from being discarded.

When the amino acid crystals are isolated from the fermentation broth in the first step, process efficiency can be increased by recycling the isolated crystals in the second step, and the amino acid content (amino acid purity) in an amino acid granule can be controlled by adjusting the dosage of amino acid crystals.

When the amino acid crystals are dissolved in the fermentation broth in the first step, a solvent may be additionally added to the fermentation broth. When a solvent is additionally added, the solvent may be added to reach the solubility of the amino acids. At this time, the solvent may be water (H₂O).

Additionally, the first step may include adding a calcium source. Specifically, a calcium source may be added to the fermentation broth before or after removing the cells from the fermentation broth in the first step.

As used herein, the term "calcium" is an essential mineral for vertebrates as it functions to make bones healthy and facilitate blood circulation. More than 99% of calcium is in bones and teeth, and the rest is present in blood and muscles. Adequate amounts of calcium maintain bone health and prevent osteoporosis. It also plays roles in preventing muscle spasms by contracting muscles and reducing the occurrence of cardiovascular disease by lowering cholesterol levels. However, when calcium is insufficient, bones are not formed properly and muscles and nerves become abnormal, so that injuries such as fractures can easily occur even with minor trauma, and the risk of osteoporosis increases. The calcium prevents diarrhea or dysentery and is also provided to animals in the form of feed additives to aid digestion and absorption, particularly in piglets.

As used herein, the term "calcium source" refers to a substance capable of providing calcium ions (Ca²⁺) without limitation. Examples of the calcium source may include calcium hydroxide (Ca(OH)₂), calcium oxide (CaO), calcium carbonate (CaCOs), calcium sulfate (CaSO₄) or calcium chloride (CaCl₂), bur are not limited thereto.

As used herein, the term "calcium hydroxide (Ca(OH)₂)" is an inorganic compound in the form of colorless crystals or white powder. Calcium hydroxide is basic when dissolved in water, and since it is inexpensive and has low toxicity, it can be used as a source of calcium ions in the present application.

As used herein, the term "calcium oxide (CaO)" is a substance that dissolves in water to generate calcium ions, playing the same role as the calcium hydroxide. Since the heat of dissolution during the dissolving process in water is relatively large, it can be provided in the form of calcium hydroxide slurry that has been previously reacted with water during the process. The reaction from calcium oxide to calcium hydroxide is as follows: CaO + H₂O → Ca(OH)₂. In order to use an equivalent amount of calcium hydroxide, that is, an equivalent molar ratio based on calcium ions, an amount obtained by multiplying calcium hydroxide by 0.76, which is the value of the molecular weight of calcium oxide for the molecular weight of calcium hydroxide, may be reacted with water and added. As shown in the above Reaction Formula, calcium oxide and water react at a molar ratio of 1:1, and thus, the amount of water to be additionally added may be obtained by subtracting the added calcium oxide from the required calcium hydroxide, or higher amount may be added so that all of the calcium oxide used can be reacted.

In the first step, the calcium source may be provided such that calcium ions are added at a molar ratio of 0.02 to 2.0, 0.05 to 2.0, 0.07 to 1.5, 0.1 to 1.0, or 0.2 to 0.6 relative to the amino acid in the fermentation broth containing amino acids, but is not limited thereto.

The calcium source may be added in the form of powder, an aqueous solution or a slurry in the calcium source addition step, but is not limited thereto.

As described above, after the first step is performed, cells are removed from the fermentation broth containing amino acids, and a fermentation filtrate is prepared. Prior to performing the granule formation of the second step for the prepared fermentation filtrate, a concentration step of concentrating the fermentation filtrate may be performed to increase the concentration of amino acids in the fermentation filtrate.

In the concentration step, the fermentation filtrate is concentrated to prepare a concentrated fermentation filtrate. Concentrating the fermentation filtrate may refer to a process of reducing the amount of solvent included in the fermentation filtrate and increasing the concentration of amino acids accordingly.

There is no limitation on the concentrating device used in the concentrating step. For example, a forced circulation concentration pipe may be used, and a paddle dryer, a slurry-drying facility, *etc.* may be used, but the device is not limited thereto.

The concentration in the concentration step may be performed at a concentration of greater than 0% (w/w) and less than or equal to 75% (w/w). The degree of concentration refers to the degree of solvent removal (weight ratio) when comparing the fermentation filtrate and the concentrated fermentation filtrate. If the degree of concentration exceeds the range, the concentrated solution becomes gel and the fluidity is significantly lowered, making it difficult to proceed to the next step. However, the above-described degree of concentration may vary depending on the type and/or operating method of equipment used in the amino acid granule preparation process. For example, the concentration may be 5% (w/w) or more and 75% (w/w) or less, 10% (w/w) or more and 70% (w/w) or less, or 20% (w/w) or more 65% (w/w) or less, but is not limited thereto.

In the concentration step, a step of introducing a calcium source may be additionally performed if necessary. When the fermentation filtrate is concentrated in the concentration step, the concentration of amino acids in the solution increases and accordingly, there is a concern that the amino acids may be precipitated in the form of crystals, and the precipitation of amino acids during the concentration process can be prevented by adding a calcium source to the concentrate. In particular, when the calcium source is not added in the first step described above, the calcium source may be added to the fermentation filtrate or the concentrated fermentation filtrate before and after concentration.

It may also include a step of pulverizing the crystals in the concentrate. This may be performed using a conventional homogenizer, but is not limited thereto. Through the pulverizing step, the average particle size of the crystals in the fermentation broth can be reduced, for example, nozzle clogging that can occur when a fluidized bed granulator is used can be prevented.

As described above, the concentration process can be performed at a relatively high concentration degree through the addition of a calcium source in the concentrate and/or crystal pulverization, and as the concentration degree increases, a larger amount of solvent is removed, and thus, the amount of water that needs to be removed can be reduced during the subsequent second step of granulation process.

Next, the second step of preparing granules from the fermentation broth obtained in the first step, in some cases, from the concentrated solution of the fermentation broth obtained in the first step, is performed.

In the second step, the "granules" are macroscopic particles, which are permanent aggregates of larger sizes made up of small particles such as powder, and may be particles with an average particle diameter of 50 µm to 5 mm, 75 µm to 4 mm or 100 µm to 3 mm.

The second step is performed to form granules, which are particles having a predetermined size, from powder or fine solid materials, and may be performed using a granulation method known in the art without limitation. For example, a mixed-type granulation method in which seeds are injected at a constant rate through a feeder into a mixed-type granulator and the fermentation broth is simultaneously supplied through a metering liquid pump to obtain granules, or a fluid bed granulation method in which a predetermined amount of seeds is introduced into a fluid bed granulator and granules are formed while injecting a predetermined amount of fermentation broth at a constant rate may be applied. In particular, a step of drying the obtained granules may be further included, but is not limited thereto.

In the second step, the mixing ratio of the seeds to the slurry solids of the fermentation broth may be 300% (w/w) to 900% (w/w). The mixing ratio of seeds to slurry solids can be used interchangeably with "seed input ratio". In some cases, the mixing ratio of the seeds may be 300% (w/w) to 850% (w/w), 350% (w/w) to 800% (w/w), or 300% (w/w) to 800% (w/w). The formation of granules can be accelerated during the process by introducing seeds at the above-described ratio. However, the seed mixing ratio may vary depending on the type of amino acids introduced during the process, the process operating conditions for granule formation in the second step, *etc.*, and is not limited to the above-described range.

As used herein, the term "seed" which is also called a crystal of a seed or a seed crystal, refers to a material used as a catalyst for crystallization or granulation of a liquid. Specifically, the seed in the present application may refer to an amino acid crystal, for example, a crystal of amino acids of the same type as the amino acids contained in the fermentation concentrate to be granulated, but is not limited thereto. When the seed is contact with the fermentation broth, the solid components present in the fermentation broth are combined with the seed to form an aggregation, thereby forming granules.

For example, the seed used at this time may have an average particle size of 150 µm to 300 µm. Specifically, a seed having an average particle size of 150 µm to 250 µm, 200 µm to 300 µm, or 200 µm to 250 µm may be used, but is not limited thereto. The particle size of the seed used may consequently affect the productivity of the production of granules according to the present application, and may be appropriately selected by those skilled in the art in consideration of the desired moisture content.

Additionally, the preparation method of the present application, prior to the second step, may further include a step of adjusting the discharged moisture rate to 5% (w/w) to 50% (w/w). In the present application, the term "discharged moisture rate" is also referred to as "mixed moisture rate", and is the ratio of moisture in the entire mixture, which can be calculated by subtracting the total solids % (w/w) from 100% (w/w) of the entire mixture. With respect to the present application, as the discharged moisture rate increases, when the fermentation broth is mixed with the seeds for subsequent granulation and added into the mixer, the recycling rate is lowered and the production rate is increased. For example, the discharged moisture rate may be adjusted to 5% (w/w) to 50% (w/w), 5% (w/w) to 45% (w/w), 6% (w/w) to 40% (w/w), 10% (w/w) to 30% (w/w), or 15% (w/w) to 30% (w/w), but is not limited thereto. However, if the discharged moisture rate is less than the above range, it may be difficult to transfer the slurry due to high viscosity unit, and if it exceeds the above range, there may be problems of overload in the downstream processes and excessive use of steam during granulation. The discharged moisture rate may have a slightly different range depending on the type of amino acids contained in the concentrate.

The discharge moisture rate may be determined by the input rate of the slurry of the fermentation broth. Specifically, as the input rate of the slurry increases, the moisture content of the granular particles may increase, and as the input rate of the slurry decreases, the moisture content of the granular particles may decrease. Since the input rate is determined according to the scale of the fermentation broth slurry, those skilled in the art can select and determine the input rate appropriately.

In addition, the preparation method of the present application may further include one or more steps selected from a step of preparing a fermentation broth; a pH adjustment step; a concentration step; a drying step; and a separation step. Each of the above steps can be performed using a method known in the art (US 2021-0094903 A1) without limitation. Furthermore, specific conditions may be appropriately changed to optimize the process, but is not limited thereto.

According to the present application, it is possible to obtain an amino acid granule from which the cells are removed through the first to second steps as discussed above. Since the amino acid granule does not contain cells, the amino acid content in the product may be at least 50%, 60%, 70%, 75%, 80%, 85%, or 90% or more. Therefore, the preparation method of the present application is suitable for providing a product with high amino acid content.

### [Advantageous Effects]

The preparation method of the present application provides an amino acid granule from which cells are removed. Since the amino acid granule provided according to the present application does not contain cells, it has higher amino acid content at the same weight compared to other amino acid granules containing cells.

### [Detailed Description of the Invention]

Hereinafter, the present application will be described in more detail by way of Examples. However, these Examples are given for illustrative purposes only, and the scope of the present application is not intended to be limited to or by these Examples

### Experimental Example 1. Preparation of Amino acid granule Containing Tryptophan

In Experimental Example 1, an amino acid granule containing tryptophan was prepared. To this end, a fermentation broth containing tryptophan (concentration 90.0 g/L, solid content 13.5% (w/w), specific gravity 1.035, purity 64.4%, cell 15%) was prepared, and amino acid crystals were isolated from the tryptophan-containing fermentation broth using a centrifuge (PTM006, Tomoe Engineering Co., Ltd., 4000/3500 RPM, feeding rate 2.1 LPM, Dam No. 1), and then the cells were isolated using a plate-type filtration membrane (Ceramic filtration system/Pure Tech, temperature 60°C, TMP 1.0, Inverter 40 Hz) to prepare a fermentation filtrate. After adding a calcium source to the fermentation filtrate, the amino acid crystals were concentrated using a 20 L concentrating tube (N-21 NS, EYELA, water bath temperature 70°C, vacuum pressure 120 Torr), and then the amino acid crystals in the concentrate were pulverized using a blender (Hanil Electric, SHMF-3600TG) to prepare a concentrated fermentation filtrate. The concentrated fermentation filtrate and the seeds generated in the process were mixed in a Ploughshare^{®} Mixer granulator (Lodige) to obtain a product.

The weight of tryptophan-containing amino acid crystals generated in the process was about 4.9 kg, the content of tryptophan in the amino acid crystals was about 2.0 kg, the moisture content in the amino acid crystals is about 57.8% (w/w), and the solid content in the amino acid crystals was about 42.2% (w/w).

In addition, the total weight of the tryptophan-containing fermentation filtrate used in the process was about 31.0 kg, the total volume was about 31.1 L, the tryptophan content in the fermentation filtrate was about 390.1 g (about 1.9 mol), the concentration of tryptophan in the fermentation filtrate was about 12.5 g/L, the pH of the fermentation filtrate was about 5.8, the solid content in the fermentation filtrate was about 2.5% (w/w) (0.8 kg),and the amount of tryptophan contained in the fermentation filtrate was about 50.3% of the total solids content.

The calcium source introduced into the process was calcium hydroxide, and the input amount was about 28.3 g (about 0.2 mol).

The calcium source was added to the fermentation filtrate, the solid content of the fermentation filtrate into which the calcium source was added was about 2.6% (796.6 g), and the amount of tryptophan contained in the fermentation filtrate into which the calcium source was added was about 49.0% of the total solid content.

Additionally, in the case of the concentrated fermentation filtrate provided by concentrating the fermentation filtrate into which the calcium source was added, the concentration was about 240.7 g/L, and the solid content was about 48.3 wt%.

### Experimental Example 1-1. Preparation of Amino acid granule with Tryptophan Purity of 84%

An amino acid granule having a tryptophan purity of 84% was prepared using the concentrated fermentation filtrate, amino acid crystals, and seeds prepared in Experimental Example 1.

In amino acid granules, the tryptophan purity was calculated by calculating the weight ratio of tryptophan to the total solid content (the total weight minus the weight occupied by moisture) contained in the amino acid granule.

The table below summarizes the input amount of concentrated fermentation filtrate added to the process, amino acid crystals, seeds, and amino acid granules prepared therefrom, solid content, moisture content, and tryptophan content of each element.

**[Table 1]**

| **Item** | **Weight (g)** | **Solid Content (g)** | **Moisture Content (g)** | **Tryptophan Content (g)** |
|---|---|---|---|---|
| **Concentrated Fermentation Filtrate** | 200.0 | 96.6 | 103.4 | 48.6 |
| **Amino Acid Crystals (TDR)** | 140.0 | 59.1 | 80.9 | 57.2 |
| Seed | 542.0 | 542.0 | 0.0 | 482.4 |
| **Amino acid granule (Tryptophan Granules)** | 882.0 | 697.7 | 184.3 | 588.1 |

As a result of preparing the amino acid granule, about 882.0 g of tryptophan granules were prepared, and the solid content of the tryptophan granules was 697.7 g, the moisture content was 184.3 g, and the tryptophan content was 588.1 g. It was analyzed that the tryptophan purity, which is the ratio of tryptophan among the solids contained in the tryptophan granules, was about 84.3%, and the moisture ratio was about 20.9%.

The classification results according to the size of tryptophan granules contained in the amino acid granule prepared in the process are as follows:

**[Table 2]**

| **Size** | **0.85 mm or more** | **0.37-0.85 mm** | **0.21-0.37 mm** | **0.21 mm or less** | **Total** |
|---|---|---|---|---|---|
| | **20 mesh** | **20-45 mesh** | **45-70 mesh** | **70 mesh or more** | |
| **Content (%)** | 84.4 | 84.5 | 83.9 | 83.5 | |
| **Weight (g)** | 4.1 | 76.1 | 176.6 | 299.1 | 555.8 |
| **Weight Ratio (%)** | 0.7 | 13.7 | 31.8 | 53.8 | 100 |

From the above experimental results, it was confirmed that the amino acid granule having high tryptophan purity could be prepared from the concentrated fermentation filtrate, amino acid crystals, and seeds after removing the cells. In addition, it was confirmed through the tryptophan granule size analysis that the content of amino acids for each particle size was uniform.

### Experimental Example 1-2. Preparation of Amino acid granule in Which Moisture Content Was Regulated

Next, in the preparation of an amino acid granule having a tryptophan purity of 84% using the concentrated fermentation filtrate, amino acid crystals, and seeds prepared in Experimental Example 1, it was confirmed whether the moisture content could be changed while maintaining the tryptophan purity in the final product.

The table below summarizes the input amount of concentrated fermentation filtrate added to the process, amino acid crystals, seeds, and amino acid granules prepared therefrom, solid content, moisture content, and tryptophan content of each element.

**[Table 3]**

| **Item** | **Weight (g)** | **Solid Content (g)** | **Moisture Content (g)** | **Tryptophan Content (g)** |
|---|---|---|---|---|
| **Concentrated Fermentation Filtrate** | 65.0 | 31.4 | 33.6 | 15.8 |
| **Amino Acid Crystals (TDR)** | 196.0 | 82.7 | 113.3 | 80.1 |
| **Seed** | 500.0 | 500.0 | 0.0 | 420.0 |
| **Amino acid granule (Tryptophan Granules)** | 761.0 | 614.1 | 146.9 | 515.8 |

As a result of preparing the amino acid granule, about 761.0 g of tryptophan granules were prepared, and the solid content of the tryptophan granules was 614.1 g, the water content was 146.9 g, and the tryptophan content was 515.8 g. It was analyzed that the tryptophan purity, which is the ratio of tryptophan among the solids contained in the tryptophan granules, was about 84.0%, and the moisture ratio was about 19.3%.

**[Table 4]**

| **Item** | **Weight (g)** | **Solid Content (g)** | **Moisture Content (g)** | **Tryptophan Content (g)** |
|---|---|---|---|---|
| **Concentrated Fermentation Filtrate** | 81.0 | 39.1 | 41.9 | 19.7 |
| **Amino Acid Crystals (TDR)** | 245.0 | 103.4 | 141.6 | 100.1 |
| **Seed** | 500.0 | 500.0 | 0.0 | 420.0 |
| **Amino acid granule** | 826.0 | 642.5 | 183.5 | 539.7 |
| **(Tryptophan Granules)** | | | | |

As a result of preparing the amino acid granule, about 826.0 g of tryptophan granules were prepared, and the solid content of the tryptophan granules was 642.5 g, the water content was 183.5 g, and the tryptophan content was 539.7 g. It was analyzed that the tryptophan purity, which is the ratio of tryptophan among the solids contained in the tryptophan granules, was about 84.0%, and the moisture ratio was about 22.2%.

However, when the moisture ratio was about 25%, it was confirmed that granulation was not performed well.

### Experimental Example 1-3. Preparation of Amino acid granule with Tryptophan Purity of About 90%

Next, it was confirmed whether amino acid granules having different tryptophan purity could be prepared using the concentrated fermentation filtrate, amino acid crystals, and seeds prepared in Experimental Example 1. Specifically, it was confirmed whether an amino acid granule having a tryptophan purity of 91% could be prepared using the same concentrated fermentation filtrate, amino acid crystals, and seeds.

The table below summarizes the input amount of concentrated fermentation filtrate added to the process, amino acid crystals, seeds, and amino acid granules prepared therefrom, solid content, moisture content, and tryptophan content of each element.

**[Table 5]**

| **Item** | **Weight (g)** | **Solid Content (g)** | **Moisture Content (g)** | **Tryptophan Content (g)** |
|---|---|---|---|---|
| **Concentrated Fermentation Filtrate Crystals (TDR)** | 111.0 | 53.6 | 57.4 | 27.0 |
| **Amino Acid** | 338.1 | 142.7 | 195.4 | 138.1 |
| **Seed** | 650.0 | 650.0 | 0.0 | 608.4 |
| **Amino acid granule (Tryptophan Granules)** | 1099.1 | 846.3 | 252.8 | 773.5 |

As a result of preparing the amino acid granule, about 1099.1 g of tryptophan granules were prepared, and the solid content of the tryptophan granules was 846.3 g, the moisture content was 252.8 g, and the tryptophan content was 773.5 g. It was analyzed that the tryptophan purity, which is the ratio of tryptophan among the solids contained in the tryptophan granules, was about 91.4%, and the moisture ratio was about 23.0%.

The classification results according to the size of tryptophan granules contained in the amino acid granule prepared in the process are as follows:

**[Table 6]**

| **Size** | **0.85 mm or more** | **0.37-0.85 mm** | **0.21-0.37 mm** | **0.21 mm or less** | **Total** |
|---|---|---|---|---|---|
| | **20 mesh** | **20-45 mesh** | **45-70 mesh** | **70 mesh or more** | |
| **Content (%)** | 88.3 | 89.2 | 90.6 | 88.7 | |
| **Weight (g)** | 12.8 | 59.8 | 161.0 | 466.5 | 700.2 |
| **Weight Ratio (%)** | 1.8 | 8.5 | 23.0 | 66.6 | 100 |

From the above experimental results, it was confirmed that the amino acid granule with higher tryptophan purity could be prepared from the concentrated fermentation filtrate, amino acid crystals, and seeds after removing the cells.

In addition, it was confirmed through the tryptophan granule size analysis that the content of amino acids for each particle size was uniform.

### Experimental Example 1-4. Preparation of Amino acid granule with Tryptophan Purity of About 70%

Next, it was confirmed whether an amino acid granule with reduced tryptophan purity could be produced using the concentrated fermentation filtrate, amino acid crystals, and seeds prepared in Experimental Example 1. Specifically, it was confirmed whether an amino acid granule having a tryptophan purity of 70% could be prepared using the same concentrated fermentation filtrate, amino acid crystals, and seeds.

The table below summarizes the input amount of concentrated fermentation filtrate added to the process, amino acid crystals, seeds, and amino acid granules prepared therefrom, solid content, moisture content, and tryptophan content of each element.

**[Table 7]**

| **Item** | **Weight (g)** | **Solid Content (g)** | **Moisture Content (g)** | **Tryptophan Content (g)** |
|---|---|---|---|---|
| **Concentrated Fermentation Filtrate** | 170.0 | 82.1 | 87.9 | 41.3 |
| **Amino Acid Crystals (TDR)** | 110.0 | 46.4 | 63.6 | 44.9 |
| **Seed** | 550.0 | 550.0 | 0.0 | 383.4 |
| **Amino acid granule (Tryptophan Granules)** | 830.0 | 678.5 | 151.5 | 469.6 |

As a result of preparing the amino acid granule, about 830.0 g of tryptophan granules were prepared, and the solid content of the tryptophan granules was 678.5 g, the moisture content was 151.5 g, and the tryptophan content was 469.6 g. It was analyzed that the tryptophan purity, which is the ratio of tryptophan among the solids contained in the tryptophan granules, was about 69.2%, and the moisture ratio was about 18.2%.

With respect to the above-described amino acid granule having a tryptophan purity of about 70%, it was confirmed whether only the moisture content could be changed while maintaining the tryptophan purity.

**[Table 8]**

| **Item** | **Weight (g)** | **Solid Content (g)** | **Moisture Content (g)** | **Tryptophan Content (g)** |
|---|---|---|---|---|
| **Concentrated Fermentation Filtrate** | 210.0 | 101.4 | 108.6 | 51.0 |
| **Amino Acid Crystals (TDR)** | 140.0 | 59.1 | 80.9 | 57.2 |
| **Seed** | 550.0 | 550.0 | 0.0 | 383.4 |
| **Amino acid granule (Tryptophan Granules)** | 900.0 | 710.5 | 189.5 | 491.5 |

As a result of preparing the amino acid granule, about 900.0 g of tryptophan granules were prepared, and the solid content of the tryptophan granules was 710.5 g, the moisture content was 189.5 g, and the tryptophan content was 491.5 g. It was analyzed that the tryptophan purity, which is the ratio of tryptophan among the solids contained in the tryptophan granules, was about 69.2%, and the moisture ratio was about 21.2%.

The classification results according to the size of tryptophan granules contained in the amino acid granule prepared in the process are as follows:

**[Table 9]**

| **Size** | **0.85 mm or more** | **0.37-0.85 mm** | **0.21-0.37 mm** | **0.21 mm or less** | **Total** |
|---|---|---|---|---|---|
| | **20 mesh** | **20-45 mesh** | **45-70 mesh** | **70 mesh or more** | |
| **Content (%)** | 69.9 | 70.1 | 71.0 | 71.2 | |
| **Weight (g)** | 32.5 | 177.0 | 175.0 | 152.0 | 536.5 |
| **Weight Ratio (%)** | 6.1 | 33.0 | 32.6 | 28.3 | |

From the above-described experimental results, it was confirmed that the amino acid granule with reduced tryptophan purity could be prepared from the concentrated fermentation filtrate, amino acid crystals, and seeds after removing the cells. Additionally, it was confirmed that it was possible to prepare amino acid granules having various moisture contents while lowering the purity.

In addition, it was confirmed through the tryptophan granule size analysis that the content of amino acids for each particle size was similar.

However, it was confirmed that granules were not formed well when the moisture content was about 15%.

### Experimental Example 2. Preparation of Amino acid granules Containing Threonine

In Experimental Example 2, amino acid granules containing threonine were prepared. To this end, a fermentation broth containing threonine (concentration 221.0 g/L, solid content 23.3% (w/w), specific gravity 1.028, purity 92.4%, cell 16.7%) was prepared, and amino acid crystals were isolated from the threonine-containing fermentation broth using a centrifuge (PTM006, Tomoe Engineering Co., Ltd., 4000/3500 RPM, feeding rate 2.1 LPM, Dam No. 1), and then the cells were isolated using a plate-type filtration membrane (Ceramic filtration system/Pure Tech, temperature 60°C, TMP 1.0, Inverter 40 Hz) to prepare a fermentation filtrate. After adding Ca(OH)₂ at a molar ratio of 0.3 to the fermentation filtrate, the amino acid crystals were concentrated using a 20 L concentrating tube (N-21NS, EYELA, water bath temperature 70°C, vacuum pressure 120 Torr), and then the amino acid crystals in the concentrate were pulverized using a blender (Hanil Electric, SHMF-3600TG) to prepare a concentrated fermentation filtrate. The seeds were prepared using a double drum dryer (DS-21, Daesung Machinery Co., Ltd., Steam pressure 0.4 bar, Feeding rate 0.5 LPM, 10 RPM).

In the preparation of amino acid granules containing threonine, a Ploughshare^{®} Mixer granulator (Lodige) (Experimental Example 2-1) and a fluidized bed granulator (FBG-3, S1 Korea Co., Ltd.) (Experimental Example 2-2) were used in the granulation process.

### Experimental Example 2-1. Preparation of Threonine-Containing Amino acid granule Using Mixer Granulator

In the preparation of amino acid granules containing threonine, a Ploughshare^{®} Mixer granulator (Lodige) was used. The composition of the feed (concentrated fermentation filtrate) used in the experiment is as follows:

**[Table 10]**

| **Item** | | **Unit** | **Amount** |
|---|---|---|---|
| **Feed (Concentrated Fermentation Filtrate)** | **Input Amount** | g | 459.4 |
| | **Ca(OH)₂ Addition** | Ratio | 0.3 |
| | **Crystallization** | - | Yes |
| | **Threonine Concentration** | g/kg | 285.0 |
| | **pH (25°C)** | - | 9.1 |
| | **Solid Content** | % | 35.0 |
| | **Threonine Purity** | % | 81.4 |

Next, the composition of seeds used in the experiment is as follows:

**[Table 11]**

| **Item** | | **Unit** | **Amount** |
|---|---|---|---|
| **Seed** | **Input Amount** | g | 1004.5 |
| | **Ca(OH)₂ Addition** | Ratio | 0.3 |
| | **Particle Size** | - | 60 mesh or less |
| | **Solid Content** | % | 0.85 |
| | **Threonine Purity** | % | 80.7 |

The results of composition analysis of the amino acid granule in the form of threonine granules prepared by adding the concentrated fermentation filtrate feed and seeds are as follows:

**[Table 12]**

| **Size (mesh No.)** | **Weight (g)** | **Ratio (%)** | **Content (%)** | **Moisture (%)** | **Purity (%)** |
|---|---|---|---|---|---|
| **12 or less** | 427.9 | 35.7 | 77.2 | 1.87 | 78.7 |
| **12-40** | 397.0 | 33.1 | 77.1 | 3.15 | 79.6 |
| **40-60** | 275.7 | 23.0 | 77.5 | 1.99 | 79.0 |
| **60 or more** | 97.2 | 8.1 | 77.9 | 2.50 | 79.9 |
| **Total** | 1,198 | 100.0 | 77.3 | 2.37 | 79.2 |

As a result of the above experiments, the amino acid granule having a threonine purity of 79.2% was obtained.

### Experimental Example 2-2. Preparation of Threonine-Containing Amino acid granule Using Fluidized Bed Granulator

In the preparation of amino acid granules containing threonine, a fluidized bed granulator (FBG-3, S1 Korea Co., Ltd.) was used. The composition of the feed (concentrated fermentation filtrate) used in the experiment is as follows:

**[Table 13]**

| **Item** | | **Unit** | **Amount** |
|---|---|---|---|
| **Feed (Concentrated Fermentation Filtrate)** | **Input Amount** | g | 4216.0 |
| | **Ca(OH)₂ Addition** | Ratio | 0.3 |
| | **Crystallization** | - | No |
| | **Threonine Concentration** | g/kg | 175.1 |
| | **pH (25°C)** | - | 9.1 |
| | **Solid Content** | % | 21.1 |
| | **Threonine Purity** | % | 80.8 |

Next, the composition of seeds used in the experiment is as follows. The seeds were prepared using a pilot drum dryer.

**[Table 14]**

| **Item** | | **Unit** | **Amount** |
|---|---|---|---|
| **Seed** | **Input Amount** | g | 200.0 |
| | **Ca(OH)₂ Addition** | Ratio | 0.3 |
| | **Particle Size** | - | 60 mesh or less |
| | **Solid Content** | % | 0.85 |
| | **Threonine Purity** | % | 81.2 |

The results of composition analysis of the amino acid granule in the form of threonine granules prepared by adding the concentrated fermentation filtrate feed and seeds are as follows:

**[Table 15]**

| **Size (mesh No.)** | **Weight (g)** | **Ratio (%)** | **Content (%)** | **Moisture (%)** | **Purity (%)** |
|---|---|---|---|---|---|
| **12 or less** | 0.0 | 0.0 | - | - | - |
| **12-40** | 806.0 | 78.7 | 80.5 | 0.57 | 80.9 |
| **40-60** | 171.5 | 16.7 | 79.9 | 0.58 | 80.4 |
| **60 or more** | 46.6 | 4.6 | 80.0 | 1.83 | 81.5 |
| **Total** | 1,024 | 100.0 | 80.4 | 0.63 | 80.9 |

As a result of the above experiments, the amino acid granule having a threonine purity of 80.9% was obtained.

As described above, it was confirmed that both a fluidized bed granulator and a mixer granulator can be used in the preparation of amino acid granules containing threonine. The threonine purity in the prepared amino acid granules was substantially the same in both cases.

### Experimental Example 3. Preparation of Amino acid granules Containing Isoleucine

In Experimental Example 3, amino acid granules containing isoleucine were prepared. To this end, a fermentation broth containing isoleucine (concentration 86.9 g/L, solid content 10.5% (w/w), specific gravity 1.030, purity 81.0%, cell 11%) was prepared, and a concentrated fermentation filtrate was prepared in the same manner as in Experimental Example 2.

In the preparation of amino acid granules containing isoleucine, a Ploughshare^{®} Mixer granulator (Lodige) (Experimental Example 3-1) and a fluidized bed granulator (FBG-3, S1 Korea Co., Ltd.) (Experimental Example 3-2) were used.

### Experimental Example 3-1. Preparation of Isoleucine-Containing Amino acid granule Using Mixer Granulator

In the preparation of amino acid granules containing isoleucine, a mixer granulator was used. The composition of the feed (concentrated fermentation filtrate) used in the experiment is as follows:

**[Table 16]**

| **Item** | | **Unit** | **Amount** |
|---|---|---|---|
| **Feed (Concentrated Fermentation Filtrate)** | **Input Amount** | g | 345.2 |
| | **Ca(OH)₂ Addition** | Ratio | 0.3 |
| | **Crystallization** | - | Yes |
| | **Isoleucine Concentration** | g/kg | 217.0 |
| | **pH (25°C)** | - | 9.1 |
| | **Solid Content** | % | 32.1 |
| | **Isoleucine Purity** | % | 67.6 |

Next, the composition of seeds used in the experiment is as follows. The seeds were prepared using a pilot drum dryer.

**[Table 17]**

| **Item** | | **Unit** | **Amount** |
|---|---|---|---|
| **Seed** | **Input Amount** | g | 516.4 |
| | **Ca(OH)₂ Addition** | Ratio | 0.3 |
| | **Particle Size** | - | 60 mesh or less |
| | **Moisture Content** | % | 2.0 |
| | **Isoleucine Purity** | % | 67.6 |

The results of composition analysis of the amino acid granule in the form of isoleucine granules prepared by adding the concentrated fermentation filtrate feed and seeds are as follows:

**[Table 18]**

| **Size (mesh No.)** | **Weight (g)** | **Ratio (%)** | **Content (%)** | **Moisture (%)** | **Purity (%)** |
|---|---|---|---|---|---|
| **12 or less** | 50.5 | 8.1 | 64.6 | 1.64 | 65.6 |
| **12-40** | 152.8 | 24.5 | 69.5 | 1.63 | 70.6 |
| **40-60** | 125.1 | 20.0 | 67.2 | 1.87 | 68.5 |
| **60 or more** | 296.4 | 47.4 | 68.5 | 1.73 | 69.7 |
| **Total** | 625 | 100.0 | 68.2 | 1.73 | 69.4 |

As a result of the above experiments, the amino acid granule having an isoleucine purity of 69.4% was obtained.

### Experimental Example 3-2. Preparation of Isoleucine-Containing Amino acid granule Using Fluidized Bed Granulator

In the preparation of amino acid granules containing isoleucine, a fluidized bed granulator was used. The composition of the feed (concentrated fermentation filtrate) used in the experiment is as follows:

**[Table 19]**

| **Item** | | **Unit** | **Amount** |
|---|---|---|---|
| **Feed (Concentrated Fermentation Filtrate)** | **Input Amount** | g | 5055.0 |
| | **Ca(OH)₂ Addition** | Ratio | 0.3 |
| | **Crystallization** | - | No |
| | **Isoleucine Concentration** | g/kg | 44.4 |
| | **pH (25°C)** | - | 9.3 |
| | **Solid Content** | % | 5.9 |
| | **Isoleucine Purity** | % | 69.2 |

Next, the composition of seeds used in the experiment is as follows. The seeds were prepared using a pilot drum dryer.

**[Table 20]**

| **Item** | | **Unit** | **Amount** |
|---|---|---|---|
| **Seed** | **Input Amount** | g | 200.0 |
| | **Ca(OH)₂ Addition** | Ratio | 0.3 |
| | **Particle Size** | - | 40-60 mesh |
| | | | 60 mesh or less |
| | **Moisture Content** | % | 2.1 |
| | **Isoleucine Purity** | % | 59.0 |

The results of composition analysis of the amino acid granule in the form of isoleucine granules prepared by adding the concentrated fermentation filtrate feed and seeds are as follows:

**[Table 21]**

| **Mesh No.** | **Weight (g)** | **Ratio (%)** | **Content (%)** | **Moisture (%)** | **Purity (%)** |
|---|---|---|---|---|---|
| 12 or less | 12.0 | 3.5 | 66.4 | 1.12 | 67.2 |
| 12-40 | 34.3 | 10.0 | 70.2 | 1.34 | 71.1 |
| 40-60 | 70.0 | 20.3 | 69.9 | 1.32 | 70.8 |
| 60 or more | 227.9 | 66.2 | 65.3 | 1.77 | 66.5 |
| Total | 344 | 100.0 | 66.8 | 1.61 | 67.9 |

As a result of the above experiments, the amino acid granule having an isoleucine purity of 67.9% was obtained.

As described above, it was confirmed that both a fluidized bed granulator and a mixer granulator can be used in the preparation of amino acid granules containing isoleucine. The isoleucine purity in the prepared amino acid granules was uniform in both cases.

### Experimental Example 4. Preparation of Amino acid granules Containing Valine

In Experimental Example 4, amino acid granules containing valine were prepared. To this end, a fermentation broth containing valine (concentration 68.4 g/L, solid content 10.1% (w/w), specific gravity 1.020, purity 66.8%, cell 5%) was prepared, and a concentrated fermentation filtrate was prepared in the same manner as in Experimental Example 2.

In the preparation of amino acid granules containing valine, a Ploughshare^{®} Mixer granulator (Lodige) (Experimental Example 4-1) and a fluidized bed granulator (FBG-3, S1 Korea Co., Ltd.) (Experimental Example 4-2) were used.

### Experimental Example 4-1. Preparation of Valine-Containing Amino acid granule Using Mixer Granulator

In the preparation of amino acid granules containing valine, a mixer granulator was used. The composition of the feed (concentrated fermentation filtrate) used in the experiment is as follows:

**[Table 22]**

| **Item** | | **Unit** | **Amount** |
|---|---|---|---|
| **Feed (Concentrated Fermentation Filtrate)** | **Input Amount** | g | 346.7 |
| | **Ca(OH)₂ Addition** | Ratio | 0.3 |
| | **Crystallization** | - | Yes |
| | **Valine Concentration** | g/kg | 225.1 |
| | **pH (25°C)** | - | 9.1 |
| | **Solid Content** | % | 28.9 |
| | **Valine Purity** | % | 77.9 |

Next, the composition of seeds used in the experiment is as follows. The seeds were prepared using a pilot drum dryer.

**[Table 23]**

| **Item** | | **Unit** | **Amount** |
|---|---|---|---|
| **Seed** | **Input Amount** | g | 651.0 |
| | **Ca(OH)₂ Addition** | Ratio | 0.3 |
| | **Particle Size** | - | 60 mesh or less |
| | **Moisture Content** | % | 2.5 |
| | **Valine Purity** | % | 75.5 |

The results of composition analysis of the amino acid granule in the form of valine granules prepared by adding the concentrated fermentation filtrate feed and seeds are as follows:

**[Table 24]**

| **Mesh No.** | **Weight (g)** | **Ratio (%)** | **Content (%)** | **Moisture (%)** | **Purity (%)** |
|---|---|---|---|---|---|
| **12 or less** | 396.5 | 54.9 | 75.0 | 0.39 | 75.3 |
| **12-40** | 182.5 | 25.3 | 72.2 | 1.25 | 73.1 |
| **40-60** | 86.0 | 11.9 | 74.2 | 1.44 | 75.3 |
| **60 or more** | 57.5 | 8.0 | 69.2 | 0.96 | 69.8 |
| **Total** | 723 | 100.0 | 73.7 | 0.78 | 74.3 |

As a result of the above experiments, the amino acid granule having a valine purity of 74.3% was obtained.

### Experimental Example 4-2. Preparation of Valine-Containing Amino acid granule Using Fluidized Bed Granulator

In the preparation of amino acid granules containing valine, a fluidized bed granulator was used. The composition of the feed (concentrated fermentation filtrate) used in the experiment is as follows:

**[Table 25]**

| **Item** | | **Unit** | **Amount** |
|---|---|---|---|
| **Feed (Concentrated Fermentation Filtrate)** | **Input Amount** | g | 4760.0 |
| | **Ca(OH)₂ Addition** | Ratio | 0.3 |
| | **Crystallization** | - | No |
| | **Valine** | g/kg | 81.6 |
| | **Concentration** | | |
| | **pH (25°C)** | - | 9.3 |
| | **Solid Content** | % | 10.5 |
| | **Valine Purity** | % | 77.7 |

Next, the composition of seeds used in the experiment is as follows. The seeds were prepared using a pilot drum dryer.

**[Table 26]**

| **Item** | | **Unit** | **Amount** |
|---|---|---|---|
| **Seed** | **Input Amount** | g | 200.0 |
| | **Ca(OH)₂ Addition** | Ratio | 0.3 |
| | **Particle Size** | - | 60 mesh or less |
| | **Moisture Content** | % | 2.5 |
| | **Valine Purity** | % | 75.5 |

The results of composition analysis of the amino acid granule in the form of valine granules prepared by adding the concentrated fermentation filtrate feed and seeds are as follows:

**[Table 27]**

| **Mesh No.** | **Weight (g)** | **Ratio (%)** | **Content (%)** | **Moisture (%)** | **Purity (%)** |
|---|---|---|---|---|---|
| 12 or less | 0.0 | 0.0 | - | - | - |
| 12-40 | 249.0 | 39.7 | 74.7 | 0.70 | 75.3 |
| 40-60 | 208.4 | 33.2 | 74.5 | 0.78 | 75.0 |
| 60 or more | 170.3 | 27.1 | 74.8 | 2.25 | 76.5 |
| Total | 628 | 100.0 | 74.6 | 1.15 | 75.5 |

As a result of the above experiments, the amino acid granule having a valine purity of 75.5% was obtained.

As described above, it was confirmed that both a fluidized bed granulator and a mixer granulator can be used in the preparation of amino acid granules containing valine. The valine purity in the prepared amino acid granules was uniform in both cases.

### Experimental Example 5. Preparation of Amino acid granules Containing Histidine

In Experimental Example 5, amino acid granules containing histidine were prepared. To this end, a fermentation broth(concentration 97.0 g/kg, solid content 15.4% (w/w), purity 63.0%, cell 13%) containing histidine was prepared, and a concentrated fermentation filtrate was prepared in the same manner as in Experimental Example 2.

In the preparation of amino acid granules containing histidine, a Ploughshare^{®} Mixer granulator (Lodige) (Experimental Example 5-1) and a fluidized bed granulator (FBG-3, S1 Korea Co., Ltd.) (Experimental Example 5-2) were used.

### Experimental Example 5-1. Preparation of Histidine-Containing Amino acid granule Using Mixer Granulator

In the preparation of amino acid granules containing histidine, a mixer granulator was used. The composition of the feed (concentrated fermentation filtrate) used in the experiment is as follows:

**[Table 28]**

| **Item** | | **Unit** | **Amount** |
|---|---|---|---|
| **Feed (Concentrated Fermentation Filtrate)** | **Input Amount** | g | 40.0 |
| | **Ca(OH)₂ Addition** | Ratio | 0.3 |
| | **Histidine Concentration** | g/kg | 195.4 |
| | **pH (25°C)** | - | 8.63 |
| | **Solid Content** | % | 29.1 |
| | **Histidine Purity** | % | 67.1 |

Next, the composition of the seeds used in the experiment is as follows. The seeds were prepared by drying at about 100°C for 2 hours using a vacuum dryer.

**[Table 29]**

| **Item** | | **Unit** | **Amount** |
|---|---|---|---|
| **Seed** | **Input Amount** | g | 380.0 |
| | **Ca(OH)₂ Addition** | Ratio | 0.3 |
| | **Particle Size** | - | 40 mesh or less |
| | **Moisture Content** | % | 2.1 |
| | **Histidine Purity** | % | 66.3 |

The results of composition analysis of the amino acid granule in the form of histidine granules prepared by adding the concentrated fermentation filtrate feed and seeds are as follows:

**[Table 30]**

| **Mesh No.** | **Weight (g)** | **Ratio (%)** | **Content (%)** | **Moisture (%)** | **Purity (%)** |
|---|---|---|---|---|---|
| **12 or less** | 113.2 | 27.3 | 65.0 | 1.64 | 66.1 |
| **12-40** | 132.3 | 31.9 | 64.8 | 1.63 | 65.9 |
| **40-60** | 76.5 | 18.5 | 66.4 | 1.87 | 67.7 |
| **60 or more** | 92.4 | 22.3 | 66.3 | 1.73 | 67.5 |
| **Total** | 414 | 100.0 | 65.5 | 1.70 | 66.6 |

As a result of the above experiments, the amino acid granule having a histidine purity of 66.6% was obtained.

### Experimental Example 5-2. Preparation of Histidine-Containing Amino acid granule Using Fluidized Bed Granulator

In the preparation of amino acid granules containing histidine, a fluidized bed granulator was used. The composition of the feed (concentrated fermentation filtrate) used in the experiment is as follows:

**[Table 31]**

| **Item** | | **Unit** | **Amount** |
|---|---|---|---|
| **Feed (Concentrated Fermentation Filtrate)** | **Input Amount** | g | 896.2 |
| | **Ca(OH)₂ Addition** | Ratio | 0.3 |
| | **Histidine Concentration** | g/kg | 195.4 |
| | **pH (25°C)** | - | 8.63 |
| | **Solid Content** | % | 29.1 |
| | **Histidine Purity** | % | 67.1 |

Next, the composition of the seeds used in the experiment is as follows. The seeds were prepared by drying at about 100°C for 2 hours using a vacuum dryer.

**[Table 32]**

| **Item** | | **Unit** | **Amount** |
|---|---|---|---|
| **Seed** | **Input Amount** | g | 80.0 |
| | **Ca(OH)₂ Addition** | Ratio | 0.3 |
| | **Particle Size** | - | 40 mesh or less |
| | **Moisture Content** | % | 2.1 |
| | **Histidine Purity** | % | 66.3 |

The results of composition analysis of the amino acid granule in the form of histidine granules prepared by adding the concentrated fermentation filtrate feed and seeds are as follows:

**[Table 33]**

| **Mesh No.** | **Weight (g)** | **Ratio (%)** | **Content (%)** | **Moisture (%)** | **Purity (%)** |
|---|---|---|---|---|---|
| 12 or less | 0.0 | 0.0 | | | 0.0 |
| 12-40 | 177.3 | 60.0 | 65.0 | 0.48 | 65.3 |
| 40-60 | 57.6 | 19.5 | 64.8 | 0.44 | 65.1 |
| 60 or more | 60.8 | 20.6 | 68.2 | 0.96 | 68.8 |
| Total | 296 | 100.0 | 65.6 | 0.57 | 66.0 |

As a result of the above experiments, the amino acid granule having a histidine purity of 66.0% was obtained

As described above, it was confirmed that both a fluidized bed granulator and a mixer granulator can be used in the preparation of amino acid granules containing histidine. The histidine purity in the prepared amino acid granules was similar in both cases.

Through the above Experimental Examples, it was confirmed that amino acid granules from which strains are removed can be prepared using various devices for various amino acids. As can be seen in the above Experimental Examples, when the amino acid granules were prepared by removing the strain, it was confirmed that it is advantageous to prepare granules with a high amino acid content as the amino acid content in the final product increases.

Additionally, it was confirmed that amino acid granules can be prepared using both the fluidized bed granulator and the mixer granulator by adjusting the input amount of seeds, concentrated fermentation filtrate, and, if necessary, amino acid crystals used in the process.

From the foregoing, a skilled person in the art to which the present application pertains will be able to understand that the present application may be embodied in other specific forms without modifying the technical concepts or essential characteristics of the present application. In this regard, the exemplary embodiments disclosed herein are only for illustrative purposes and should not be construed as limiting the scope of the present application. On the contrary, the present application is intended to cover not only the exemplary embodiments but also various alternatives, modifications, equivalents, and other embodiments that may be included within the spirit and scope of the present application as defined by the appended claims.

## Claims

1. A method for preparing an amino acid granule, comprising:
a first step of preparing a fermentation filtrate by removing cells from a fermentation broth containing amino acids; and
a second step of forming granules from the fermentation filtrate.

2. The method of claim 1, wherein the amino acid is an amino acid having a water solubility of greater than 0 g/100 g and less than or equal to 20 g/100 g at 25°C.

3. The method of claim 1, wherein the amino acid is at least one selected from the group consisting of valine, tryptophan, threonine, isoleucine, leucine, methionine, histidine, and phenylalanine.

4. The method of claim 1, wherein the first step comprises concentrating the fermentation filtrate.

5. The method of claim 1, wherein the first step further comprises separating amino acid crystals from the fermentation broth,
wherein the isolated amino acid crystals are provided to the second step.

6. The method of claim 1, wherein the first step further comprises dissolving amino acid crystals present in the fermentation broth and/or fermentation filtrate.

7. The method of claim 1, wherein the first step further comprises adding a calcium source to the fermentation broth and/or fermentation filtrate.

8. The method of claim 7, wherein the calcium source is provided so that calcium ions are added in a molar ratio of 0.02 to 2.0 relative to amino acids.

9. The method of claim 1, wherein the method further comprises resupplying at least a portion of the amino acid seeds obtained in the second step to the granule formation process.

10. Amino acid granules having an amino acid content of 50% or more and no cells.

11. The method of claim 7 wherein the amino acid granule comprises calcium in a molar ratio of 0.02 to 2.0 relative to the amino acids in the granule.
